Europäisches Patentamt

**European Patent Office**

**Office européen des brevets**

(11) Numéro de publication: **0 225 667 B1**

## FASCICULE DE BREVET EUROPEEN

(45) Date de publication de fascicule du brevet: **10.02.93**  (51) Int. Cl.⁵: **A61B 8/06**, G01S 15/89

(21) Numéro de dépôt: **86202109.4**

(22) Date de dépôt: **27.11.86**

(54) **Appareil d'exploration par échographie ultrasonore de milieux en mouvement et notamment d'écoulements sanguins ou d'organes tels que le coeur.**

(30) Priorité: **03.12.85 FR 8517851**
**25.03.86 FR 8604225**

(43) Date de publication de la demande:
**16.06.87 Bulletin 87/25**

(45) Mention de la délivrance du brevet:
**10.02.93 Bulletin 93/06**

(84) Etats contractants désignés:
**BE DE ES FR GB NL SE**

(56) Documents cités:
**EP-A- 0 081 045**
**EP-A- 0 092 841**
**DE-B- 2 551 946**

(73) Titulaire: **LABORATOIRES D'ELECTRONIOUE PHILIPS**
**3, Avenue Descartes**
**F-94450 Limeil-Brévannes(FR)**

(84) Etats contractants désignés:
**FR**

(73) Titulaire: **N.V. Philips' Gloeilampenfabrieken**
**Groenewoudseweg 1**
**NL-5621 BA Eindhoven(NL)**

(84) Etats contractants désignés:
**BE DE ES GB NL SE**

(72) Inventeur: **Bonnefous, Odile Société Civile S.P.I.D.**
**209, rue de l'Université**
**F-75007 Paris(FR)**
Inventeur: **Pesque, Patrick Société Civile S.P.I.D.**
**209, rue de l'Université**
**F-75007 Paris(FR)**

(74) Mandataire: **Landousy, Christian et al**
**Société Civile S.P.I.D. 156, Boulevard Haussmann**
**F-75008 Paris(FR)**

Rank Xerox (UK) Business Services

**Description**

La présente invention concerne un appareil d'exploration de milieux en mouvement par échographie ultrasonore comprenant un étage d'émission de signaux impulsionnels ultrasonores périodiques vers le milieu à explorer, un étage de réception et de traitement des signaux échographiques renvoyés par ledit milieu, un circuit de suppression des échos fixes pour l'élimination de ceux desdits signaux renvoyés qui correspondent dans le milieu exploré à des objets fixes et la sélection des signaux correspondant aux objets mobiles, un dispositif de mémorisation, de conversion de balayage et de codage de couleur, et un dispositif de visualisation de paramètres d'écoulement associés localement à chaque point du milieu exploré.

Depuis plusieurs années, des systèmes Doppler ultrasonores à ondes pulsées sont couramment utilisés pour mesurer en un point donné les vitesses d'écoulements sanguins, ou du moins la projection de telles vitesses sur l'axe du faisceau émis par les transducteurs ultrasonores. Plus récemment sont apparus des appareils permettant d'obtenir en temps réel la distribution des vitesses d'écoulements sur le trajet parcouru par l'onde ultrasonore et, plus encore, sur le plan de coupe obtenu en balayant le transducteur. La plupart de ces systèmes exploitent le décalage de fréquence ou le décalage de phase du signal rétrodiffusé par les cibles mobiles pour en déduire la vitesse axiale des écoulements sanguins.

La demande de brevet européen EP-A-92841, par exemple, a trait à un tel appareil. L'appareil décrit dans ladite demande utilise la mesure du décalage de phase entre les échos successifs rétrodiffusés par les cibles en mouvement, en réponse à une excitation récurrente. Cependant, l'utilisation de la méthode du décalage de fréquence ou de phase en Doppler pulsé est limitée notamment par le phénomène dit "d'aliasing", ou de détermination biaisée, qui empêche l'estimation de vitesses de valeurs absolues supérieures à une certaine limite définie par

$$V_{lim} = \frac{c}{4} \cdot \frac{F}{f_c}$$

où c représente la vitesse de propagation, F la fréquence de récurrence de l'excitation, et $f_c$ la fréquence centrale du signal échographique. Ce phénomène est décrit en particulier dans l'ouvrage "Doppler Ultrasound and Its Use in Clinical Measurement", P. Atkinson and J.P. Woodcock, Academic Press, 1982, chapitre III, paragraphe 3.3d.

De plus, les appareils de mise en oeuvre de cette méthode connue utilisant le décalage de phase sont limités par une relation d'incertitude liant la résolution axiale $\Delta z$ et la précision de la mesure de vitesse $\frac{\Delta V}{V}$ à la longueur d'onde $\lambda$ :

$$\frac{\Delta V}{V} \cdot \Delta z = \frac{\lambda}{2} \qquad (1)$$

Cette relation, citée dans le chapitre II, paragraphe 2.3a, de l'ouvrage mentionné plus haut (relation 2.30), impose donc un compromis entre la résolution axiale et la précision de la mesure de vitesse, ce qui est incompatible avec l'établissement précis d'un profil de vitesse ou d'une image d'écoulements sanguins.

Le but de l'invention est de proposer un appareil tel que défini dans le préambule mais dans lequel cette limitation de la gamme de mesure des vitesses disparaît, et dans lequel la précision de la mesure de vitesse augmente avec la résolution axiale.

A cet effet, l'appareil selon l'invention est caractérisé en ce qu'il comprend un circuit d'estimation des paramètres d'écoulement par mise en corrélation desdits signaux associés à des objets mobiles pour deux émissions de signaux périodiques successives et un circuit discriminateur comprenant lui-même :
- des moyens de sélection d'un signal de différence entre des signaux associés à des objets mobiles pour deux émissions de signaux périodiques successives ;
- des moyens de calcul d'énergie locale, en fonction de la partie dudit signal de différence qui correspond à chaque région du milieu examiné ;
- des moyens de comparaison de l'énergie ainsi calculée à un seuil prédéterminé, pour éliminer les paramètres d'écoulement estimés en association à une région déterminée si cette énergie est inférieure audit seuil.

Dans cette structure selon l'invention, il est donc prévu un type de traitement de signal dans le domaine temporel, avec lequel on interprète la rétrodiffusion des signaux ultrasonores non plus en termes de décalage de fréquence ou de phase mais en termes de décalage temporel progressif des signaux échographiques à la suite de chaque envoi de signaux impulsionnels. Ladite structure utilise en effet un nouveau principe d'estimation en temps réel des paramètres d'écoulement qui repose sur la formulation temporelle des signaux ultrasonores rétrodiffusés par les cibles en mouvement. Les signaux échographiques successivement obtenus en réponse aux excitations récurrentes du transducteur fixe peuvent se mettrent sous la forme :

$$e_{i+1}(t) = e_i(t - \tau(t)) \qquad (2)$$

où i est le rang de la ligne échographique et où $\tau(t)$ traduit le décalage temporel induit par le déplacement des cibles entre deux tirs. Le décalage est lié à la vitesse axiale locale $V_z(t)$ (projection sur l'axe Z de propagation de l'onde ultrasonore) par la relation :

$$\tau(t) = 2 \cdot \frac{V_z(t)}{c} \cdot T \qquad (3)$$

où c est la vitesse de propagation des ultrasons, et T la période de récurrence des excitations. La localisation de la vitesse d'écoulement est traduite par la relation classique en échographie liant le temps t à la profondeur Z :

$$z = \tfrac{1}{2} \cdot ct \qquad (4)$$

L'usage de fonctions d'intercorrélation permet alors de mesurer les décalages temporels entre signaux (voir l'article "The generalized correlation method for estimation of time delay", C.H. KNAPP et G.C. CARTER, IEEE Transactions on Acoustics, Speech and Signal Processing, Vol. ASSP-24 N°4, 1976). Appliqué au cas de l'échographie, il vient pour la fonction d'intercorrélation entre $e_i$ et $e_{i+1}$ :

$$f_i(t_0, u) = \int_{t_0}^{t_0 + w} e_i(t) \cdot e_{i+1}(t + u)\, dt \qquad (5)$$

La détermination de la composante axiale de la vitesse d'écoulement, moyennée sur la cellule de résolution définie par la fenêtre temporelle de longueur W et la largeur du faisceau ultrasonore, s'obtient par la recherche du décalage temporel u pour lequel la fonction d'intercorrélation $f_i$ est maximale. Ce décalage temporel qui rend $f_i$ maximale est égal à $\tau(t_0)$, valeur moyenne des décalages $\tau(t)$ sur la fenêtre $(t_0, t_0 + w)$, ce qui permet la mesure de la vitesse locale moyenne d'écoulement par la relation (3).

D'autre part, on montre que la variance locale de la vitesse d'écoulement $\sigma^2(t_0)$ dans la cellule de résolution est proportionnelle à la quantité définie par :

$$\left( 1 - \frac{f_i(t_0, \tau(t_0))}{E_i(t_0)} \right)^{\frac{1}{2}} \qquad (6)$$

où $E_i(t_0)$ est l'énergie du signal échographique dans la fenêtre $(t_0, t_0 + W)$ :

$$E_i(t_0) = \int_{t_0}^{t_0 + w} e_i^2(t) \cdot dt \qquad (7)$$

Des expressions précédentes il apparaît clairement que le principe de l'invention basé sur la détermination des paramètres d'écoulement par fonction d'intercorrélation remédie aux limitations des réalisations antérieures. En effet, l'estimation du décalage temporel $\tau$ peut être conduite sans ambiguïté quelle que soit sa valeur. En conséquence, il n'existe plus de limitation sur la gamme de vitesses mesurables. D'autre part, le traitement étant basé sur la mesure du décalage temporel des échos successifs dû au changement de position des cibles, il est clair qu'une bonne résolution axiale, autorisant la détermination précise de la position des cibles, permet de même la mesure fine de leur déplacement et donc de leur vitesse, ce qui n'était pas possible avec les réalisations antérieures compte tenu de la relation d'incertitude mentionnée ci-dessus.

La demande de brevet européen EP-A-81045 décrit également un appareil d'examen par échographie ultrasonore utilisable pour l'analyse d'écoulements sanguins. Mais cet appareil d'une part utilise lui aussi la méthode de décalage de fréquence, avec les limitations déjà signalées, et d'autre part comprend des moyens d'estimation des paramètres d'écoulement qui sont sans commune mesure avec la structure du circuit d'estimation décrit ici et des moyens de détection d'enveloppe qui, à l'évidence, n'ont aucune caractéristique ni aucun effet communs avec ceux du circuit discriminateur présent dans l'appareil selon l'invention.

Dans l'appareil selon l'invention, qui peut comprendre une voie de traitement analogique traditionnelle pour la visualisation de plans de coupe explorés selon le principe de l'échographie classique, le circuit de suppression des échos fixes comprend, de préférence, successivement un convertisseur analogique-numérique, commandé par des signaux de commande du rythme d'échantillonnage à une fréquence $f = 1/\Delta t$, et un soustracteur recevant sur sa première entrée de signe déterminé directement la sortie du convertisseur et sur sa deuxième entrée de signe contraire cette même sortie mais retardée d'un nombre entier de périodes $\Delta t$ d'échantillonnage dans un circuit à retard, et délivrant un signal dit de différence. Le signal de différence ainsi délivré est utilisé à la fois dans le circuit suivant d'estimation de paramètres d'écoulement et dans le circuit discriminateur.

Le circuit d'estimation de paramètres d'écoulement comprend ici d'une part un circuit d'intercorrélation qui, à partir desdits signaux de différence relatifs à deux lignes échographiques successives, délivre un nombre déterminé de valeurs de fonction de corrélation, et d'autre part un circuit d'interpolation qui, à partir desdites valeurs, délivre des paramètres représentatifs de la vitesse des milieux explorés le long de l'axe de propagation des ondes ultrasonores. Le circuit d'intercorrélation comprend alors en général un nombre impair $(2I + 1)$ de voies en parallèle composées chacune d'un corrélateur commandé par lesdits signaux de commande à la fréquence d'échantillonnage F. Lesdits $(2I + 1)$ corrélateurs reçoivent sur une première entrée directement ledit signal de différence délivré par le circuit de suppression des échos fixes et sur une deuxième entrée ce même signal mais retardé dans des lignes à retard dont les retards prennent $(2I + 1)$ valeurs distinctes $T - I \Delta t$, $T-(I-1) \Delta t$, $T-(I-2) \Delta t$, ..., $T - \Delta t$, $T$, $T+ \Delta t$,..., $T+(I-2) \Delta t$, $T+(I-1) \Delta t$, $T+I \Delta t$, et délivrent $(2I + 1)$ valeurs de fonction de corrélation.

A ces corrélateurs, qui sont de préférence des corrélateurs 1 bit, il est particulièrement approprié d'associer un circuit d'interpolation linéaire. Des circuits de calcul de moyenne peuvent avantageusement être prévus dans chacune des $(2I +1)$ voies en parallèle du circuit d'intercorrélation, en sortie des corrélateurs, avant le circuit d'interpolation linéaire.

On peut noter par ailleurs que la relation de récurrence (2) énoncée plus haut est applicable également aux signaux obtenus en ne prenant en compte que le signe des signaux échographiques correspondant aux lignes échographiques, ce qui donne :

$$\begin{aligned} s_{i+1}(t) &= \text{signe de } (e_{i+1}(t)) \\ &= \text{signe de } (e_{i+1}(t-\tau(t))) \\ &= s_i(t-\tau(t)) \end{aligned} \tag{8}$$

L'information contenue dans ces signaux réduits au signe $S_i(t)$ est en effet suffisante pour estimer localement des vitesses moyennes, car les fronts montants ou descendants d'un tel signal $s_{i+1}(t)$ se révèlent être des répliques, retardées de $\tau(t)$, du signal précédent $s_i(t)$. On dispose donc d'une estimation $\hat{\tau}$ de $\tau(t)$ pour chaque front montant ou descendant du signal $s_i$ en mesurant le décalage temporel entre ce front, montant ou descendant, et le front correspondant du signal suivant $s_{i+1}$. Une information est ainsi disponible toutes les demi-périodes du signal échographique.

Une multiplication 1 bit s'avère d'ailleurs être particulièrement adaptée à la mesure de $\tau$ : lorsque les signaux sont différents, le résultat de la multiplication 1 bit est 0, alors qu'il est égal à 1 si ces signaux sont identiques. De cette manière, la fonction $f_i(t_0)$ définie ci-dessous :

4

$$f_i(t_0) = \frac{1}{w} \int_{t_0}^{t_0 + w} s_i(t) \cdot s_{i+1}(t).dt \qquad (9)$$

où $t_0$ et $t_0 + w$ encadrent une fenêtre temporelle de longueur w, vérifie la relation :

$$f_i(t_0) = 1 - 2 \, \mu_0 \, |\hat{\tau}(t_0)|$$
$$= 1 - 4 \, \upsilon_0 \, T \, |\hat{V}(t_0)|/c \qquad (10)$$

où $\hat{V}$ est la vitesse estimée et $\nu_0$ la fréquence centrale. En fait, ce calcul ne permet d'obtenir que l'amplitude de la vitesse et non son sens qui représente pourtant une information très importante pour le clinicien.

Pour lever cette ambiguïté quant au sens de la vitesse, il faut considérer que $f_i(t_0)$ est la valeur prise en 0 par la fonction de corrélation locale, définie par :

$$C(t_{0,u}) = \frac{1}{w} \int_{t_0}^{t_0 + w} s_i(t) \cdot s_{i+1}(t+u).dt \qquad (11)$$

Cette fonction a les propriétés suivantes. D'une part, si la vitesse est positive, $\tau(t)$ est supérieur à 0 et l'expression :

$$C(t_{0,u}) = \frac{1}{w} \int_{t_0}^{t_0 + w} s_i(t) \cdot s_i(t+u-\tau).dt$$

est une fonction croissante au voisinage de u = 0, tandis que cette même expression $C(t_{0,u})$ est une fonction décroissante au voisinage de u = 0 si la vitesse est au contraire négative. D'autre part, au voisinage du pic de corrélation, $C(t_{0,u})$ est triangulaire.

Ainsi, le calcul de la pente A de $C(t_{0,u})$ au voisinage de u = 0 permet de déterminer le sens de la vitesse ainsi que la valeur du pic de corrélation $C_{max}$. Cette valeur est en effet utilisable pour estimer la variance $\sigma$ de la vitesse selon la relation :

$$\sigma = (1 - C_{max})^{\frac{1}{2}} \qquad (12)$$

$C_{max}$ est en fait calculé de la manière suivante :
$C_{max} = C(t_0,0) + |A| \, |\hat{\tau}|$
$= C(t_0,0) + |A|(1-C(t_0,0))/2 \, \nu_0$
ou encore :

$$C_{max} = (1-|A|/2 \, \upsilon_0).C(T_0,0) + \left|\frac{A}{2\upsilon_0}\right| \qquad (13)$$

Une unité de traitement comprenant un corrélateur 1 bit à trois voies de calcul et une unité de calcul fournira donc une estimation des paramètres V et $\sigma_V$.

Dans une variante de réalisation, le circuit d'estimation de paramètres d'écoulement comprend d'une part un circuit d'intercorrélation qui, à partir desdits signaux de différence relatifs à deux lignes échographiques successives, délivre trois valeurs de fonction de corrélation, et d'autre part une unité de calcul qui, à partir desdites valeurs, délivre des paramètres représentatifs de la vitesse des milieux explorés le long de l'axe de propagation des ondes ultrasonores. Le circuit d'intercorrélation comprend dans ce cas trois voies en parallèle composées chacune d'un corrélateur 1 bit commandé par lesdits signaux de commande à la

5

fréquence d'échantillonnage F, lesdits corrélateurs recevant sur une première entrée directement ledit signal de différence délivré par le circuit de suppression des échos fixes et sur une deuxième entrée ce même signal mais retardé dans des lignes à retard dont les retards prennent trois valeurs distinctes T - $\Delta t$, T, T + $\Delta t$, et délivrant trois valeurs de fonction de corrélation. Là encore, il est avantageux de prévoir des circuits de calcul de moyenne dans chacune des trois voies en parallèle, en sortie des corrélateurs.

Dans l'une ou l'autre de ces réalisations, le circuit discriminateur comprend des moyens de sélection d'un signal de différence entre signaux associés à des objets mobiles pour deux émissions successives. Plus précisément, ce circuit comprend, de préférence, successivement un circuit d'élévation au carré du signal de différence délivré par le circuit de suppression des échos fixes, un sommateur de calcul de l'énergie locale du signal de différence, et un circuit de validation, ou non, des signaux de sortie du circuit d'estimation de paramètres d'écoulement selon la valeur calculée de ladite énergie locale, ce calcul étant obtenu à l'aide de moyens de calcul d'énergie locale qui utilisent la partie de signal de différence qui correspond à chaque région du milieu examiné, en vue d'une comparaison de l'énergie calculée à un seuil et, si celle-ci est inférieure au seuil, de l'élimination des paramètres associés à une région déterminée. Il est en outre avantageux de prévoir dans ce circuit discriminateur un autre circuit de validation, ou non, des paramètres d'écoulement selon la valeur de l'un au moins de ces paramètres.

Les particularités et avantages de l'invention apparaîtront maintenant de façon plus précise dans la description qui suit et dans les dessins annexés, donnés à titre d'exemples et dans lesquels :

- la figure 1 montre un exemple de réalisation préférentiel de l'appareil selon l'invention, et les figures 2 et 3 des exemples particuliers de réalisation respectivement de l'étage d'émission et du circuit de suppression des échos fixes de cet appareil ;
- la figure 4 montre un premier mode de réalisation du circuit d'estimation de paramètres d'écoulement, et la figure 5 met en évidence, dans le cas de ce mode de réalisation, le principe de fonctionnement du circuit d'interpolation, lorsque celui-ci est linéaire et pour un calcul de $(2l + 1) = 13$ valeurs de fonction de corrélation ;
- la figure 6 montre un deuxième mode de réalisation du circuit d'estimation de paramètres d'écoulement, et la figure 7 met en évidence, dans le cas de ce mode de réalisation, le principe de fonctionnement de l'unité de calcul ;
- la figure 8 représente un exemple de réalisation du circuit discriminateur de l'appareil de la figure 1.

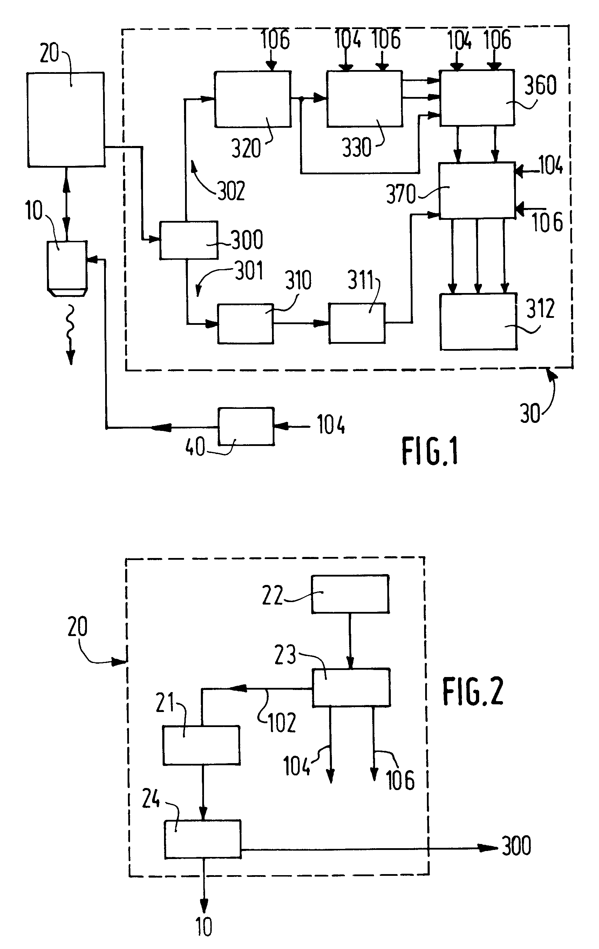

L'appareil représenté sur la figure 1 comprend, de façon classique, un transducteur ultrasonore 10 associé d'une part à un étage d'émission 20 et d'autre part à un étage 30 de réception et de traitement, ainsi qu'à un dispositif 40 de commande de balayage mécanique du transducteur. A la place de ce transducteur pourrait bien entendu être utilisé un réseau de transducteurs, associé alors à un dispositif de commande de balayage électronique.

Dans la réalisation décrite de façon plus détaillée sur la figure 2, l'étage d'émission 20 comprend un générateur 21 de signaux électriques d'excitation, envoyés vers le transducteur 10 qui les convertit en trains périodiques de signaux impulsionnels ultrasonores. Cette émission est commandée par des signaux d'horloge disponibles sur une connexion 102 et délivrés à une fréquence de récurrence F déterminée -de l'ordre de 5 kilohertz par exemple- par un séquenceur comprenant successivement un oscillateur 22, ici de fréquence 32 mégahertz, et un diviseur de fréquence 23. Ce diviseur délivre les signaux d'horloge sur la connexion 102, ainsi que d'autres signaux de commande respectivement sur des connexions 104 et 106, à 1 kilohertz et 16 mégahertz dans l'exemple ici décrit. Les signaux de commande présents sur la connexion 104 commandent en particulier le dispositif 40, pour le balayage du transducteur. Un séparateur 24 des étages d'émission 20 et de réception et de traitement 30 est inséré entre le transducteur 10, le générateur 21, et ledit étage 30, et évite l'aveuglement des circuits de réception par les signaux d'émission.

L'étage de réception et de traitement 30 comprend, en sortie du séparateur 24, un amplificateur haute fréquence 300 incluant la compensation de gain en fonction de la profondeur et suivi de deux voies de traitement 301 et 302 prévues en parallèle. La voie 301, de type traditionnel, comprend ici, en série, un détecteur d'enveloppe 310, un amplificateur de compression logarithmique 311, un dispositif de mémorisation et de conversion de balayage 370 incluant aussi une fonction de codage de couleur, et un dispositif 312 de visualisation. Cette voie 301 permet l'obtention, sous forme d'images en échelle de gris, de plans de coupe des milieux explorés selon le principe de l'échographie classique. La voie 302 comprend en série un circuit 320 de suppression des échos fixes, un circuit 330 d'estimation de paramètres d'écoulement, un circuit discriminateur 360, le dispositif de mémorisation, de conversion de balayage et de codage de couleur 370, et le dispositif de visualisation 312.

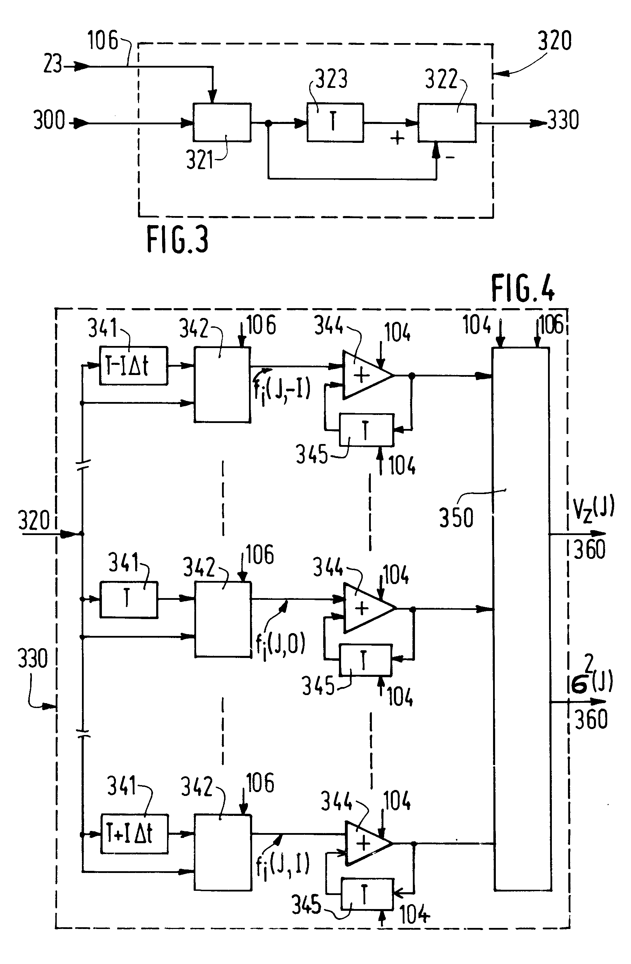

Le circuit numérique 320 de suppression des échos fixes comprend lui-même, dans l'exemple de réalisation représenté sur la figure 3, un convertisseur analogique-numérique 321 dont la sortie est reliée d'une part directement à l'entrée négative d'un soustracteur 322 et d'autre part à l'entrée positive de ce soustracteur par l'intermédiaire d'un circuit à retard 323. Le retard apporté par ce dernier circuit 323 pourrait être de plusieurs périodes T = 1/F, mais il est avantageux que ce retard soit le plus faible possible et égal à T. Ce circuit 320 est prévu pour l'élimination de tous les échos fixes, en particulier de ceux dont l'apparition est provoquée par la réflexion des ondes ultrasonores contre les parois des vaisseaux où se produisent les écoulements étudiés. Cette présence d'échos fixes est gênante du fait de leur amplitude bien plus élevée (de l'ordre de +40 dB dans le cas des écoulements sanguins) que celle des signaux utiles, les signaux rétrodiffusés par les cibles mobiles. Le circuit 320 est également commandé, par l'intermédiaire de la connexion 106, par le diviseur de fréquence 23 du séquenceur qui lui fournit le signal de commande d'échantillonnage à la fréquence de 16 mégahertz.

Dans un premier mode de réalisation, représenté sur la figure 4, le circuit 330 d'estimation de paramètres d'écoulement comprend un circuit d'intercorrélation et un circuit d'interpolàtion. A partir des signaux de différence entre deux lignes échographiques successives d'échantillons $d_i(t)$, $d_{i+1}(t)$, etc... (où i représente le rang de ce signal),successivement fournis par le circuit 320 de suppression des échos fixes, le circuit d'intercorrélation délivre des valeurs de fonction de corrélation, ici en nombre impair (2I + 1). A partir de ces (2I + 1) valeurs, le circuit d'interpolation délivre des paramètres caractérisant les différents écoulements rencontrés le long de l'axe de propagation de l'onde ultrasonore. Ces paramètres sont ici la composante axiale de la vitesse locale moyenne $V_z$ et la variance locale $\sigma^2$ de celle-ci, le mot local étant ici employé au sens de la localisation en profondeur le long dudit axe Z de propagation.

Le circuit d'intercorrélation comprend dans ce mode de réalisation (2I + 1) corrélateurs 342 qui, sur une première entrée, reçoivent chacun directement la sortie $d_{i+1}(t)$ du circuit 320 de suppression d'échos fixes et, sur une deuxième entrée, reçoivent cette même sortie du circuit 320, mais retardée par des lignes à retard 341 et correspondant donc au signal précédent $d_i(t)$. De plus, chacune de ces lignes 341 présente, pour permettre le calcul des (2I + 1) valeurs de la fonction de corrélation, un retard distinct prenant (2I + 1) valeurs de T - I $\Delta$t à T + I $\Delta$t, où $\Delta$t représente la période d'échantillonnage imposée par la connexion 106.

Ce calcul, effectué en parallèle, des (2I + 1) valeurs de la fonction de corrélation utilise K échantillons successifs des deux signaux d'entrée des corrélateurs. Les groupes de K échantillons définissent des fenêtres temporelles successives de longueur K $\Delta$t se décalant progressivement au rythme de la fréquence imposée par la connexion 106. La fonction de corrélation est définie par une expression du type :

$$f_i(J,P) = \sum_{k=1}^{k=K} d_i((k+J)\,\Delta t) \cdot d_{i+1}((k+J+P)\,\Delta t) \quad (14)$$

dans laquelle :
- J détermine le début de la fenêtre temporelle de longueur K $\Delta$t ;
- P représente le décalage temporel introduit entre $d_i$ et $d_{i+1}$ et pour lequel on calcule la valeur de la fonction de corrélation (P variant de -I à +I par pas de 1) ;
- i représente le rang de la différence entre deux lignes échographiques successives $e_i$ et $e_{i+1}$.

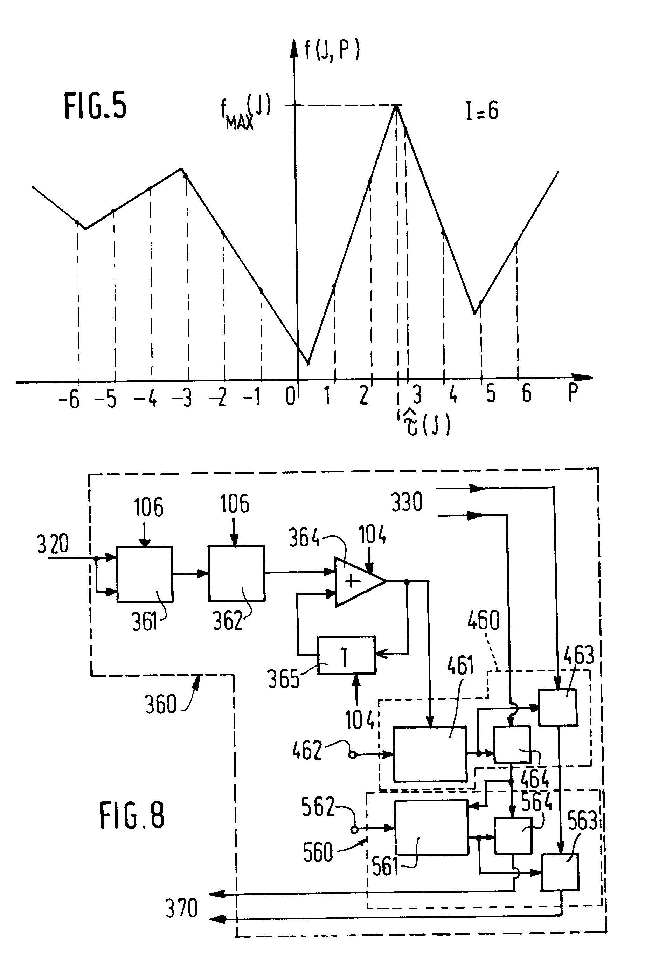

Les corrélateurs 342, commandés par l'intermédiaire de la connexion 106 de sortie du diviseur de fréquence 23 du séquenceur, sont de préférence des corrélateurs 1 bit qui présentent plusieurs avantages : d'une part, la mise en oeuvre de la méthode de corrélation est simplifiée, d'autre part le pic principal de corrélation obtenu a une forme triangulaire qui se prête bien à une interpolation linéaire, et enfin des corrélateurs 1 bit en circuit intégré sont à l'heure actuelle largement disponibles sur le marché et relativement peu coûteux (par exemple le corrélateur TDC 1023 de la société TRW, La Jolla, CA 92038, USA). Lorsque la réalisation décrite comprend ces corrélateurs 1 bit, le circuit d'interpolation 350 est donc, en général, un circuit d'interpolation linéaire. La figure 5, montre, pour I = 6 par exemple, les 13 valeurs obtenues de la fonction de corrélation, ainsi que la valeur interpolée correspondant à l'amplitude du sommet du pic principal de corrélation. Sur la figure 4, la fonction de corrélation est notée $f_i(J, -I)$ pour le premier corrélateur, $f_i(J, -(I-1))$ pour le suivant (non représenté),... $f_i(J,0)$ pour le (I+1)ième, etc..., et $f_i(J,I)$ pour le (2I + 1)ième corrélateur.

Le circuit d'interpolation peut être constitué par une unité de calcul programmée, incluant un microprocesseur, ou de préférence par une unité de calcul câblée. Ce circuit d'interpolation fonctionne de la façon suivante : on recherche dans un premier temps la valeur maximale parmi les (2I + 1) valeurs de la fonction de corrélation, on y associe les deux valeurs de fonction de corrélation adjacentes, et ces trois valeurs permettent la reconstitution du pic principal de corrélation, de forme isocèle. L'abscisse $\hat{\tau}(J)$ du pic principal de corrélation donne accès à la vitesse locale $V_z$ à la profondeur

$$z_0 = \frac{cJ \ \Delta t}{2}$$

par multiplication selon la relation :

$$V_Z(z_0) = c . \frac{\hat{\tau}(J)}{2T} \qquad (15)$$

et l'amplitude $f_{MAX}$ de ce pic donne accès à la variance $\sigma^2(z_0)$ par opération selon la relation :

$$\sigma^2(z_0) = A(1 - \frac{f_{MAX}(J)}{K}) \qquad (16)$$

où A est un facteur de proportionalité.

Entre les $(2I + 1)$ corrélateurs et les $(2I + 1)$ entrées correspondantes du circuit d'interpolation 350 peuvent être prévus, et le sont ici de préférence, $(2I + 1)$ circuitsde calcul de moyenne qui sont en fait des accumulateurs comprenant chacun un additionneur 344 et une ligne à retard 345 de retard T (ou un multiple de T). Ces circuits de calcul de moyenne permettent d'accumuler les valeurs de fonction de corrélation sur N lignes échographiques successives et d'en effectuer la moyenne avant envoi vers le circuit d'interpolation. Les additionneurs 344 et les lignes à retard 345 sont reliés au séquenceur (22, 23) par l'intermédiaire de la connexion 104, pour leur remise à zéro à intervalles N x T réguliers. La fréquence de ces remises à zéro imposées par la connexion 104 est celle des signaux présents sur la connexion 102, divisée par N.

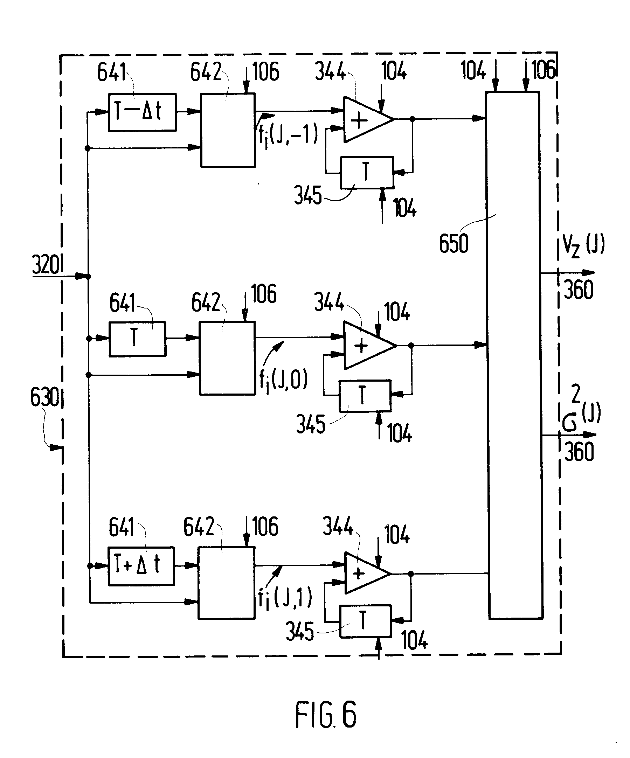

Dans un deuxième mode de réalisation, représenté sur la figure 6, le circuit d'estimation de paramètres d'écoulement, maintenant référencé 630, comprend ici un circuit d'intercorrélation et une unité de calcul 650. A partir des signaux de différence entre deux lignes échographiques successives d'échantillons $d_i(t)$, $d_{i+1}(t)$, etc... (où i représente le rang de ce signal) successivement fournis par le circuit 320 de suppression des échos fixes, le circuit d'intercorrélation délivre trois valeurs de fonction de corrélation. A partir de ces trois valeurs, l'unité de calcul délivre des paramètres caractérisant les différents écoulements rencontrés le long de l'axe de propagation de l'onde ultrasonore. Ces paramètres sont ici la composante axiale de la vitesse locale moyenne $V_Z$ et la variance locale $\sigma^2$ de celle-ci, le mot local étant ici employé au sens de la localisation en profondeur le long dudit axe Z de propagation.

Plus précisément, le circuit d'intercorrélation 630 comprend trois corrélateurs 642 qui, sur une première entrée, reçoivent chacun directement la sortie $d_{i+1}(t)$ du circuit 320 de suppression d'échos fixes et, sur une deuxième entrée, reçoivent cette même sortie du circuit 320, mais retardée par des lignes à retard 641 et correspondant donc au signal précédent $d_i(t)$. De plus, chacune de ces lignes 641 présente, pour permettre le calcul des trois valeurs de la fonction de corrélation, un retard distinct prenant trois valeurs T - $\Delta t$, T, T + $\Delta t$, où $\Delta t$ représente la période d'échantillonnage imposée par la connexion 106.

Ce calcul, effectué en parallèle, des trois valeurs de la fonction de corrélation utilise K échantillons successifs des deux signaux d'entrée des corrélateurs. Les groupes de K échantillons définissent des fenêtres temporelles successives de longueur K $\Delta t$ se décalant progressivement au rythme de la fréquence imposée par la connexion 106. La fonction de corrélation est définie comme précédemment par l'expression (14). Les corrélateurs 642, commandés par l'intermédiaire de la connexion 106 de sortie du diviseur de fréquence 23 du séquenceur, sont des corrélateurs 1 bit. L'unité de calcul 650 peut être une unité de calcul programmée, incluant un microprocesseur, ou de préférence une unité de calcul câblée. Cette unité de calcul dispose de trois valeurs de la fonction de corrélation :

$C_1 = C(t_0, -\Delta t) \qquad (17)$

$C_2 = C(t_0, 0) \qquad (18)$

$$C_3 = C(t_0, \Delta t) \qquad (19)$$

Le signe de la pente de la fonction de corrélation, auquel correspond le sens de la vitesse, est déterminé par :

$$s = \text{signe}\,(C_3 - C_1) \qquad (20)$$

En utilisant la relation (7), la vitesse est donnée par l'expression :

$$\hat{V} = s \cdot \frac{C_1 - C_2}{4\,\upsilon_0 T} \qquad (21)$$

La valeur A de la pente en 0 de la fonction de corrélation est obtenue selon le tableau ci-après :

|  | $C_2 \geqslant 0,5$ | $C_2 < 0,5$ |
|---|---|---|
| $C_3 - C_1 \geqslant 0$ | $A = (C_2 - C_1)/\Delta t$ | $A = (C_3 - C_2)/\Delta t$ |
| $C_3 - C_1 < 0$ | $A = (C_3 - C_2)/\Delta t$ | $A = (C_2 - C_1)/\Delta t$ |

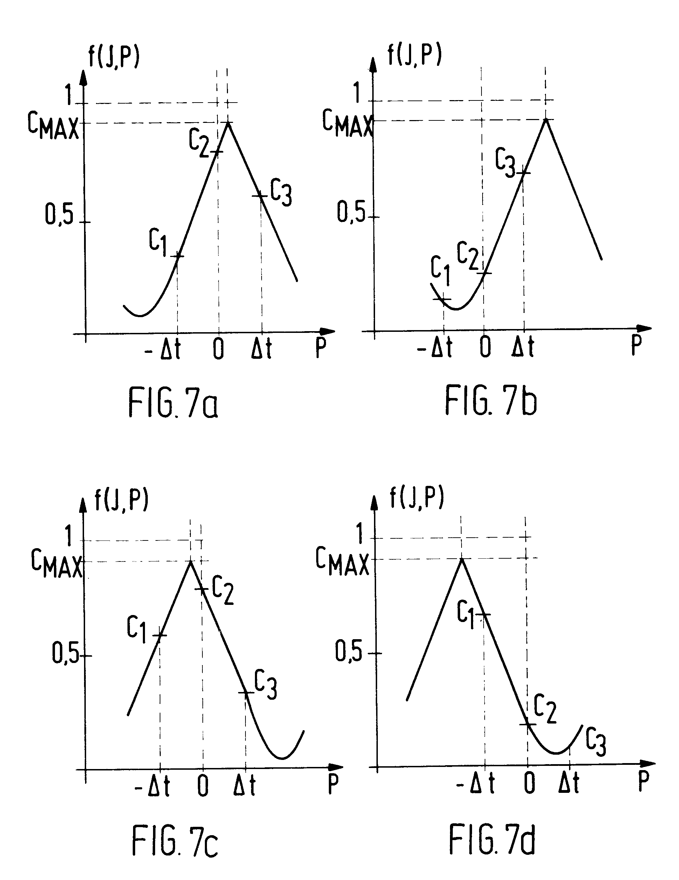

Ce tableau fait l'inventaire, en association avec les figures 7a à 7d correspondantes, des quatre types de configuration possible décrivant les valeurs $C_1$, $C_2$, $C_3$.

On en déduit la valeur $C_{MAX}$ du pic de corrélation :

$$C_{MAX} = \left(1 - \frac{|A|}{2\upsilon_0}\right) \cdot C_2 + \frac{|A|}{2\upsilon_0} \qquad (22)$$

ainsi que la variance :

$$\sigma = (1 - C_2)^{\frac{1}{2}} \cdot \left(1 - \frac{|A|}{2\,\upsilon_0}\right)^{\frac{1}{2}}$$

Entre les trois corrélateurs et les trois entrées correspondantes de l'unité de calcul 650, on prévoit de préférence, comme précédemment, trois circuits de calcul de moyenne. Ces circuits sont là encore des accumulateurs comprenant chacun un additionneur 344 et une ligne à retard 345 de retard T (ou un multiple de T). Ces circuits de calcul de moyenne permettent d'accumuler les valeurs de fonction de corrélation sur N lignes échographiques successives et d'en effectuer la moyenne avant envoi vers l'unité de calcul. Comme vu ci-dessus, les additionneurs 344 et les lignes à retard 345 sont reliés au séquenceur (22, 23) par l'intermédiaire de la connexion 104, pour leur remise à zéro à intervalles N x T réguliers, et la fréquence de ces remises à zéro imposées par la connexion 104 est celle des signaux présents sur la connexion 102, divisée par N.

Quel que soit le mode de réalisation du circuit d'estimation des paramètres d'écoulement, les signaux de sortie de ce circuit 330 ou 630 sont alors validés, ou non, par le circuit discriminateur 360, puis les valeurs ainsi confirmées sont envoyées vers le dispositif de visualisation 312 par l'intermédiaire du dispositif de codage de couleur 370.

La présence du circuit discriminateur 360 est indispensable. En effet, en dehors des zones d'écoulement, le signal de sortie du circuit 320 de suppression des échos fixes est essentiellement du bruit. Le résultat alors fourni par le circuit 330 ou 630 d'estimation des paramètres de l'écoulement, qui traite ce bruit, n'est pas l'indication d'une vitesse nulle, et il devient nécessaire de valider, ou non, ce résultat. Le circuit 360, représenté sur la figure 8, comprend à cet effet en série un multiplieur 361, qui reçoit le signal de sortie $d_i$ du circuit 320 de suppression des échos fixes et élève au carré le signal de différence ainsi prélevé, un sommateur 362 qui permet, en fonction de la partie du signal de différence qui correspond à chaque région du milieu examiné, le calcul de l'énergie locale de ce signal de différence selon la formule :

$$E_i(J) = \sum_{k=1}^{k=K} d^2((k+J)\,\Delta t) \qquad (23)$$

un circuit (364, 365) de calcul de moyenne qui, comme dans le cas des circuits 344 et 345, est un accumulateur comprenant un additionneur 364 et une ligne à retard 365 de retard T (ou un multiple de T) et permet d'effectuer la moyenne de l'énergie locale pour toute une série de signaux de différence, par exemple la moyenne sur N tirs, c'est-à-dire pour (N-1) différences, selon l'expression :

$$E(J) = \sum_{i=1}^{i=N-1} E_i(J) \qquad (24)$$

avant d'envoyer la valeur ainsi obtenue vers un circuit de validation 460.

Ce circuit de validation 460 comprend un comparateur 461 recevant d'une part sur une première entrée la sortie de l'accumulateur (364, 365), ou directement celle du sommateur 362 dans le cas où le circuit de calcul de moyenne ne serait pas prévu, et d'autre part sur une deuxième entrée 462 une tension de référence formant seuil, la sortie du comparateur étant ici au niveau logique 0 ou 1 selon que la tension reçue sur sa première entrée est respectivement inférieure ou supérieure au seuil de référence. Deux multiplieurs 463 et 464, qui reçoivent respectivement les signaux de sortie $V_z$ et $\sigma^2$ du circuit 330 ou 630 sur leur première entrée transmettent ces deux signaux, notés désormais $V'_z$ et $\sigma'^2$, sur leur sortie respective ou transmettent simplement des valeurs nulles selon que le signal de validation délivré sur leur deuxième entrée par le comparateur 461 est 1 ou 0 respectivement. En effet, en dehors des zones d'écoulement vrais, l'énergie moyenne calculée en sortie du circuit (364, 365) est celle du bruit seul, et peut être mesurée seule, a priori, en l'absence d'excitation, en vue de déterminer la valeur appropriée du seuil. Au contraire, en présence de signaux rétrodiffusés par les cibles en mouvement, l'énergie moyenne du signal dv est supérieure à celle du bruit seul, ce qui autorise la validation des signaux délivrés par le circuit 330 ou 630 d'estination des paramètres de l'écoulement.

Dans une variante de réalisation de ce circuit discriminateur 360, le signal de variance lui-même, par exemple, peut être utilisé comme signal de discrimination. En effet, comme d'un tir à l'autre le bruit est décorrélé, si la variance calculée est grande, c'est parce qu'on se trouve en présence de bruit seul. Au contraire, en présence de signaux rétrodiffusés, la variance est nettement plus faible et, là encore, la présence, dans un circuit de validation 560, d'un comparateur 561, recevant sur une première entrée le signal de sortie $\sigma'^2$ du multiplieur 462 et sur une deuxième entrée 562 une tension de référence constituant un seuil, et de deux multiplieurs 563 et 564 permet de valider ou non, comme précédemment, les signaux appelés alors $V''_z$ et $\sigma''^2$ en sortie de ces multiplieurs. Bien entendu, au lieu d'être situé, comme indiqué sur la figure 8, en aval des multiplieurs 463 et 464 du circuit de validation 460, ce nouveau circuit de validation 560 pourrait être placé en amont du premier circuit de validation, les deux signaux de vitesse et de variance éventuellement validés par lui-même étant alors envoyés sur les entrées correspondantes des multiplieurs 463 et 464.

A la suite des N excitations commandées par le signal 102 et ayant permis la mesure des paramètres d'écoulement ou de déplacement des structures situées sur l'axe du transducteur, un déplacement électronique ou mécanique dudit transducteur est commandé par le signal de commande de balayage 104. Le procédé d'estimation est alors réinitialisé dans une autre direction d'observation.

Les deux sorties du circuit discriminateur 360 sont envoyées vers le dispositif de mémorisation, conversion de balayage et codage de couleur 370, par lequel transite également, avant visualisation, le signal de sortie de l'amplificateur 311 de la voie de traitement 301. Un tel dispositif est décrit par exemple dans la demande de brevet européen EP-A-0100094. La figure 3 de ce document montre en effet, entre les bornes A, B, C et $E_R$, $E_G$, $E_B$, un exemple des circuits qui peuvent être mis en place dans le cas de la présente invention, la borne A recevant le signal échographique classique et les bornes B et C les paramètres caractéristiques du milieu en mouvement examiné. Ce dispositif 370 et le dispositif 312 permettent alors la visualisation en temps réel des écoulements ou des déplacements superposés à l'image de réflectivité échographique classique.

**Revendications**

1. Appareil d'exploration de milieux en mouvement par échographie ultrasonore comprenant un étage d'émission de signaux impulsionnels ultrasonores périodiques vers le milieu à explorer, un étage de réception et de traitement des signaux échographiques renvoyés par ledit milieu, un circuit de suppression des échos fixes pour l'élimination de ceux desdits signaux renvoyés qui correspondent dans le milieu exploré à des objets fixes et la sélection des signaux correspondant aux objets mobiles, un dispositif de mémorisation, de conversion de balayage et de codage de couleur, et un dispositif de visualisation de paramètres d'écoulement associés localement à chaque point du milieu exploré, caractérisé en ce qu'il comprend un circuit (330) d'estimation des paramètres d'écoulement par mise en corrélation desdits signaux associés à des objets mobiles pour deux émissions de signaux périodiques successives et un circuit discriminateur (360) comprenant lui-même :
   - des moyens de sélection d'un signal de différence entre des signaux associés à des objets mobiles pour deux émissions de signaux périodiques successives ;
   - des moyens de calcul d'énergie locale, en fonction de la partie dudit signal de différence qui correspond à chaque région du milieu examiné ;
   - des moyens de comparaison de l'énergie ainsi calculée à un seuil prédéterminé, pour éliminer les paramètres d'écoulement estimés en association à une région déterminée si cette énergie est inférieure audit seuil.

2. Appareil selon la revendication 1, caractérisé en ce que ladite énergie calculée et comparée est l'énergie moyenne de toute une série de signaux de différence.

3. Appareil selon l'une des revendications 1 et 2, caractérisé en ce que le circuit d'estimation de paramètres opère l'estimation de la vitesse locale dans le milieu exploré, et en ce que le circuit discriminateur comprend également des moyens de comparaison de ladite vitesse locale à un seuil prédéterminé, pour éliminer les paramètres d'écoulement estimés localement si cette vitesse est inférieure audit seuil.

4. Appareil selon l'une des revendications 1 à 3, caractérisé en ce que le circuit d'estimation de paramètres d'écoulement comprend d'une part un circuit d'intercorrélation qui, à partir desdits signaux de différence relatifs à deux lignes échographiques successives, délivre un nombre déterminé de valeurs de fonction de corrélation, et d'autre part un circuit d'interpolation qui, à partir desdites valeurs, délivre des paramètres représentatifs de la vitesse des milieux explorés le long de l'axe de propagation des ondes ultrasonores.

5. Appareil selon l'une des revendications 1 à 3, caractérisé en ce que le circuit d'estimation de paramètres d'écoulement comprend d'une part un circuit d'intercorrélation qui, à partir desdits signaux de différence relatifs à deux lignes échographiques successives, délivre trois valeurs de fonction de corrélation, et d'autre part une unité de calcul qui, à partir desdites valeurs, délivre des paramètres représentatifs de la vitesse des milieux explorés le long de l'axe de propagation des ondes ultrasonores.

6. Appareil selon l'une des revendications 1 à 5, caractérisé en ce qu'il comprend également des moyens de suppression locale des paramètres d'écoulement lorsque la vitesse locale estimée dans ladite région concernée est inférieure à un seuil prédéterminé.

**Claims**

1. An apparatus for scanning moving objects by means of ultrasonic echography, which apparatus comprises a stage for the periodic transmission of ultrasonic pulse signals to the object to be scanned, a stage for receiving and processing echographic signals returned by said object, a circuit for suppressing fixed echoes in order to eliminate those returned signals which correspond to fixed objects within the object being scanned and to select the signals corresponding to the moving objects, a storage, scan conversion and colour encoding device, and a device for the display of flow parameters associated locally with each point of the object scanned, characterized in that it comprises a circuit (330) for estimating flow parameters by correlation of said signals associated with moving objects for two successive emissions of periodic signals, and also comprises a discriminator circuit (360) which

11

itself comprises:
- means for selecting a difference signal between signals associated with moving objects for two successive emissions of periodic signals;
- means for calculating the local energy as a function of the part of said difference signal which corresponds to each region of the object examined;
- means for comparing the energy thus calculated with a predetermined threshold in order to eliminate the estimated flow parameters associated with a given region if said energy is below said threshold.

2. An apparatus as claimed in Claim 1, characterized in that said calculated and compared energy is the mean energy of an entire series of difference signals.

3. An apparatus as claimed in any one of the Claims 1 and 2, characterized in that the parameter estimation circuit performs the estimation of the local velocity in the object scanned, the discriminator circuit also comprising means for comparing said local velocity with a predetermined threshold in order to eliminate the locally estimated flow parameters if said velocity is below said threshold.

4. An apparatus as claimed in any one of the Claims 1 to 3, characterized in that the circuit for estimating flow parameters comprises on the one hand a cross-correlation circuit which supplies, on the basis of said difference signals relating to two successive echographic lines, a given number of correlation function values, and on the other hand an interpolation circuit which supplies, on the basis of said values, parameters which are representative of the velocity of the objects scanned along the axis of propagation of the ultrasonic waves.

5. An apparatus as claimed in any one of the Claims 1 to 3, characterized in that the circuit for estimating flow parameters comprises on the one hand a cross-correlation circuit which supplies, on the basis of said difference signals relating to two successive echographic lines, three correlation function values, and on the other hand an arithmetic unit which supplies, on the basis of said values, parameters which are representative of the velocity of the objects scanned along the axis of propagation of the ultrasonic waves.

6. An apparatus as claimed in any one of the Claims 1 to 6, characterized in that it also comprises means for locally suppressing flow parameters when the local estimated velocity in said relevant region is below a predetermined threshold.

**Patentansprüche**

1. Gerät zur Ultraschallechographie von bewegenden Gegenständen, mit einer Ausgabestufe für periodische pulsierende Ultrasehallsignale nach dem zu untersuchenden Gebiet, mit einer Stufe zum Empfangen und Bearbeiten der echographischen Signale aus diesem Gebiet, mit einer Unterdrückungsschaltung der Festechos für die Beseitigung dieser ausgesandten Signale, die im untersuchten Gebiet Festgegenständen entsprechen und für die Wahl der beweglichen Gegenständen entsprechenden Signale, mit einer Speicher-, Abtastumsetz- und Farbcodiereinrichtung und eine Visualisiereinrichtung für die jedem Punkt des untersuchten Gebiets stellenweise zugeordneten Strömungsparameter, dadurch gekennzeichnet, daß es eine Schätzungsschaltung (330) zur Schätzung der Strömungsparameter enthält, in der die Schätzung durch die Korrelation der den beweglichen Gegenständen zugeordneten Signal für zwei aufeinanderfolgende Emissionen periodischer Signale erfolgt, und eine Diskriminatorschaltung (360) mit folgenden Elementen enthält:
- Wählmittel zum Wählen eines Unterschiedssignals zwischen den beweglichen Gegenständen zugeordneten Signalen für zwei aufeinanderfolgende Emissionen periodischer Signale;
- Berechnungsmittel für die Berechnung der örtlichen Energie in Abhängigkeit von dem Teil des Unterschiedssignals, der jedem Abschnitt des untersuchten Gebiets entspricht;
- Vergleichsmittel für die Vergleichung der auf diese Weise berechneten Energie mit einer vorgegebenen Schwelle zum Beseitigen der in Zusammenhang mit einem bestimmten Bereich geschätzten Strömungsparameter wenn diese Energie niedriger als die Schwelle ist.

2. Gerät nach Anspruch 1, dadurch gekennzeichnet, daß die berechnete und vergtichene Energie die mittlere Energie einer ganzen Reihe von Unterschiedssignalen ist.

**3.** Gerät nach einem der Ansprüche 1 und 2, dadurch gekennzeichnet, daß die Schätzungsschaltung der Parameter die Schätzung der örtlichen Geschwindigkeit im untersuchten Gebiet bewirkt, und daß die Diskriminatorschaltung ebenfalls Vergleichsmittel für die örtliche Geschwindigkeit mit einer vorgegebenen Schwelle enthält, um die örtlich geschätzten Strömungsparameter zu beseitigen, wenn diese Geschwindigkeit niedriger als die Schwelle ist.

**4.** Gerät nach einem der Ansprüche 1 bis 3, dadurch gekennzeichnet, daß die Schätzungsschaltung der Strömungsparameter einerseits eine Interkorrelationsschaltung, die, ausgehend von den Unterschiedssignalen bezüglich zwei aufeinanderfolgender echographischer Linien, eine bestimmte Anzahl von Korrelationsfunktionswerten liefert, und andererseits eine Interpolationsschaltung enthält, die, ausgehend von diesen Werten, die Geschwindigkeit der untersuchten Gegenständen entlang der Fortpflanzungsachse der Ultraschallwellen darstellende Parameter liefert.

**5.** Gerät nach einem der Ansprüche 1 bis 3, dadurch gekennzeichnet, daß die Schätzungsschaltung der Strömungsparameter einerseits eine Interkorrelationsschaltung, die, ausgehend von den Unterschiedssignalen bezüglich zwei aufeinanderfolgender echographischer Linien, drei Korrelationswerte liefert, und andererseits eine Berechnungseinheit enthält, die, ausgehend von diesen Werten, die Geschwindigkeit der untersuchten Teilen entlang der Fortpflanzungsachse der Ultraschallwellen darstellende Parameter liefert.

**6.** Gerät nach einem der Ansprüche 1 bis 5, dadurch gekennzeichnet, daß es ebenfalls Unterdrückungsmittel für die örtliche Unterdrückung von Strömungsparametern enthält, wenn die geschätzte örtliche Geschwindigkeit im betreffenden Bereich unter einer vorgegebenen Schwelle liegt.

FIG.1

FIG.2

FIG.3

FIG.4

FIG.5

$f(J,P)$

$f_{MAX}(J)$

$I=6$

-6 -5 -4 -3 -2 -1 0 1 2 3 4 5 6 P

$\hat{\tau}(J)$

FIG.8

320

106

106

364

104

361

362

365

360

104

330

460

461

463

462

464

564

563

562

560

561

370

FIG. 6

FIG. 7a

FIG. 7b

FIG. 7c

FIG. 7d